# EUROPEAN PATENT APPLICATION

(11) **EP 3 369 378 A2**
(43) Date of publication of application: **05.09.2018**
(21) Application number: 18156225.7
(22) Date of filing: 12.02.2018
(51) Int. Cl.: A61B 6/00, A61B 90/17, A61N 5/10

(54) **BREAST SUPPORT DEVICE FOR RADIOTHERAPY**

(30) Priority: 13.02.2017 US 201762458214 P; 14.12.2017 US 201715841377
(71) Applicant: Taipei Medical University, Taipei 11031 (TW); National Taipei University of Technology, Taipei 10608 (TW)
(72) Inventor: Long-Sheng, Lu, Taipei 11031 (TW); Kuo-Hsiung, Tseng, Taipei 10608 (TW); Der-Chi, Tien, Taipei City 11581 (TW); Meng-Yun, Chung, New Taipei City 22054 (TW)
(74) Representative: Zaboliene, Reda

(57) **Abstract**

A breast support device for radiation therapy includes a cuplike body with a through aperture. The cuplike body has a base portion and a compression portion connected continuously forming a concave inner surface, the compression portion is extended from the base portion, the concave inner surface have contours that fit over the female patient's breast as patient been in a predetermined posture. The through aperture is formed on the cuplike body to receive a portion of the female patient's breast through, it has a contour curve defined by a cutting plane and the contours of the patient's breast.

## Description

### TECHNICAL FIELD

The present invention relates to a medical device, and more particularly, to a breast support device for radiotherapy.

### BACKGROUND

There are many choices for breast cancer treatment. Usually, it is treated with surgery, which may be followed by chemotherapy or radiation therapy. Radiation therapy can focus on treating cancer cells inside the breast that may still stick around after surgery, which is an effective way to destroy these cancer cells. Radiation therapy is relatively easy to tolerate and its side effects are mostly limited within the treated area.

External beam radiation is one of common types of radiation therapy to destroy cancer cells in the breast. To treat a patient, the radiation beam is delivered from a radiation source, normally it is from a machine outside the body, to the area with cancer cells in the breast. Before treatments start, correct angles for aiming the radiation beams and the proper dose are determined by taking careful measurements by a radiotherapy treatment planning system. Some ink marks are made on skin as a guide to focus the radiation on the right area. Most radiation treatments are in supine position, which means lying horizontally with the face and torso facing up, as opposed to the prone position, which is face down.

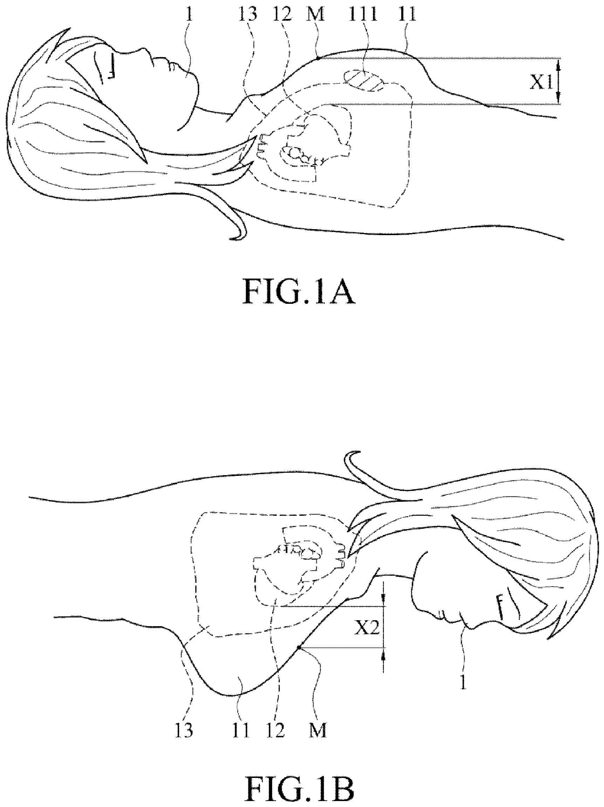

FIG. 1A shows a female patient 1 in a supine position during the radiation treatment. While patient in the supine position, a target area 111 (area with cancer cells) in a breast (or breast tissue) 11 is pulled downwards by gravity, which may cause the radiation beams to reach or pass through patient's heart 12 and lung 13. Exposure to the radiation may damage the heart 12 and the lung 13.

Referring to FIG. 1B, to protect the patient's heart 12 and the lung 13 from radiation, the patient may be treated with radiation therapy in a comparative prone position. The prone position may pull the other breast, which is not treated with radiotherapy, may be squeezed and makes the patient 1 uncomfortable. The setup in prone position is relatively complex than in the supine position.

For reducing the chance that the radiation beams pass through patient's heart 12 and lung 13, a prone position may be considered. However, drawback or risk still exists while being in the prone position, because the patient's heart 12 and lung 13 are also been pulled downwardly by gravity. In such case, the patient's heart 12 and lung 13 may still be closer to the target area 111.

When the patient 1 is in the supine position, the breast 11 is collapsed and a measured minimum distance between a reference point M of the breast 11 and the heart 12 is X1. The reference point M may be a mark made on any spot of the breast 11. When the female patient 1 is in the prone position, a measured minimum distance between the reference point M of the breast 11 and the heart 12 is X2. The distance X2 may be substantially the same as or slightly greater than the distance X1. However, difference between the distance X2 and the distance X1 wouldn't be obvious because the patient's heart 12 and lung 13 along with the breast 11 are all pulled downwardly by gravity. Therefore, in the prone position is not very helpful in separating the patient's heart 12 and lung 13 from the breast 11, as compared to the supine position. Especially, in the situation that the patient's cancer cells located inside the breast 11, to treat the patient in the prone position may have no advantages.

### SUMMARY OF THE INVENTION

As a patient takes a radiation treatment in a prone position, the patient has to lie face down on a stage with an opening allowing an ill breast to pass through. The other breast having no need to be treated with radiotherapy may be squeezed by the stage. In such case, the patient may feel uncomfortable. In considering that a patient receives a radiation treatment in a supine position, the setup of treatment in the supine position is relatively simple and the patient will feel more comfortable. However, as mentioned earlier, a patient without wearing any assistant device on, no matter what positions the patient take in during the radiation treatment, the target area with cancer cells in the breast could be very close to the patient's periphery organs, such as heart and lung. Under these circumstances, the patient's heart and lung could get irradiated by the radiation beams since at least part of the heart and the lung may also be in the path of the radiation beams. Exposure to the radiation can cause damages in the heart, the lung, and organs/tissues in or adjacent to the exposed area.

In this invention, a breast support device for radiation therapy has been proposed to correct the mentioned drawbacks.

The present invention discloses a breast support device for radiation therapy, the breast support device includes a cuplike body with a through aperture. The cuplike body has a base portion and a compression portion connected continuously forming a concave inner surface, the compression portion is extended from the base portion, the concave inner surface has contours that fit over the female patient's breast as the female patient been in a predetermined posture. The through aperture is formed on the upper portion of the compression portion to receive a portion of the female patient's breast through, it has a contour curve defined by a cutting plane and the contours of the patient's breast.

According to the present invention, the breast support device can support a breast of a female patient while the female subject lies supinely for a radiation treatment. A target area with cancer cells in the breast of the female patient can maintain its position away from the heart and lung based upon the compression and support by the breast support device. As a result, affection of patient's heart and lung due to radiation can be minimized.

### BRIEF DESCRIPTION OF THE DRAWINGS

The components, characteristics and advantages of the present invention may be understood by the detailed descriptions of the preferred embodiments outlined in the specification and the drawings attached:
FIG. 1A illustrates a female patient in a supine position according to the prior art;
FIG. 1B illustrates a female patient in a prone position according the prior art;
FIG. 2 illustrates a top view of a breast support device according to the present invention;
FIG. 3A-B illustrate top views of a breast support device according to the present invention;
FIG. 4 illustrates a bottom view of a breast support device according to the present invention;
FIG. 5 illustrates a bottom view of a breast support device according to the present invention;
FIG. 6 illustrates a female patient wearing a breast support device according to the present invention;
FIG. 7 illustrates a block diagram of a model processing system for forming a breast support device model according to the present invention;
FIG. 8 illustrates a flow chart of steps for forming a breast support device model according to the present invention;
FIG. 9 illustrates a perspective view of a female patient being scanned in a predetermined posture by a 3D scanning device for forming a breast support device model according to the present invention;
FIG. 10A-G illustrates the breast supporting device model at various stages, which correspond to a perspective view of steps shown in FIG. 8 according to the present invention;
FIGS. 11A-11C illustrate cross-sectional views of a 3D model being processed according to the present invention; and
FIG. 12 illustrates a line chart showing a dosimetric performance relative to the hearts of female patients with and without breast support devices under radiation treatments according to the present invention.

### DETAILED DESCRIPTION

Some preferred embodiments of the present invention will now be described in greater detail. However, it should be recognized that the preferred embodiments of the present invention are provided for illustration rather than limiting the present invention. In addition, the present invention can be practiced in a wide range of other embodiments besides those explicitly described, and the scope of the present invention is not expressly limited except as specified in the accompanying claims.

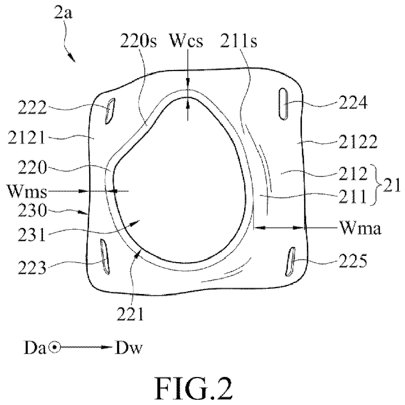

FIG. 2 illustrates a top view of a breast support device 2a according to the present invention. The breast support device 2a includes a body 21. The body 21 is composed of a base portion 212 and a compression portion 211. The compression portion 211 is connected with the base portion 212 and is extended from the base portion 212 along an axial direction Da of the body 21. The axial direction Da is regarded as a direction perpendicular to the plane of the top view of FIG. 2 or parallel to the normal of the base portion 212. The body 21 is manufactured by a material suitable for 3D printing. For example, the material can be, but not limited to, PLA (Polylactic Acid), ABS (Acrylonitrile Butadiene Styrene), TPE (Thermoplastic Elastomer), transparent TPE or similar materials suited for 3D printing. The body 21 includes relatively solid material (being solid enough to compress and shape the breast) to support the breast, such as PLA, ABS, TPE, and/or TPE. The body 21 has mechanical properties list as follow. For example, it has a tensile properties that can bear the stress during the treatments, can sustain a force ranged from approximately 450 kgf to 700 kgf, has a tensile strength ranged from approximately 4 kgf/mm² to 7 kgf/mm², has yield strength from approximately 1 kgf/mm² to approximately 4 kgf/mm², and has elongation percentage ranged from approximately 490 % to 500%.

The base portion 212 and the compression portion 211 have a thickness ranged from substantially 2 to 15 mm. In one preferred embodiment, the thickness of the base portion 212 and the compression portion 211 is ranged from 3 to 10 mm. A through aperture 221 is formed by removing a portion of the compression portion 211 through the guides of a cutting plane. A first cutting side 220 is defined by the cutting plane. A width of the annular surface 220s, i.e. Wcs, can be varied at different locations depending on the orientation of the cutting plane and the morphology of the outer surface 211s of the compression portion 211. The size of the through aperture 221, depending on patient's physical characters, can be ranged from 3 to 45 centimeters. A second opening 231 actually is form on the opposite side of the breast support device 2a relative the through aperture 221, it size is defined roughly by the size of the breast support device 2a. The aforementioned through aperture 221 refers to the resulting opening after removing the portion of the compression portion 211. The through aperture shape is one of the close loops of the breast cross-sections, and the close loop will not be a circle.

The breast support device 2a has a concave inner surface and it is usually fitted to a female patient's breast contours. The location of the through aperture 221 can be roughly defined by a distance Wms and Wma shown in FIG. 2. Wms is the shortest distance between the edge of the outer contour of the annular surface 220s and the device edge in the midsternal side 2121; Wma is the shortest distance between the edge of the outer contour of the annular surface 220s and the device edge in the midaxially side 2122. The contours refer to the shape of the breast, especially its surface or the shape formed by its outer edge. The image of a female patient's chest formed or taken by a three-dimensional (3D) scanning device contains geometrical data of the female patient's chest to form a 3D model, therefore, for example, a pluralities of lines on the 3D model or image taken by the scanning device contains lines each of which is the points of equal height or depth joined together, but each line's height or depth is different from the height or depth of other lines, in a way that shows high and low areas of breast shape. Another example is a more general definition, a plurality of lines can be defined from a plurality of parallel planes with any angle Θ, each of which cutting the 3D model. The angle Θ is defined by the normal of the parallel planes and the axial axis of the 3D model, which can be ranged from 0 to 90 degrees.

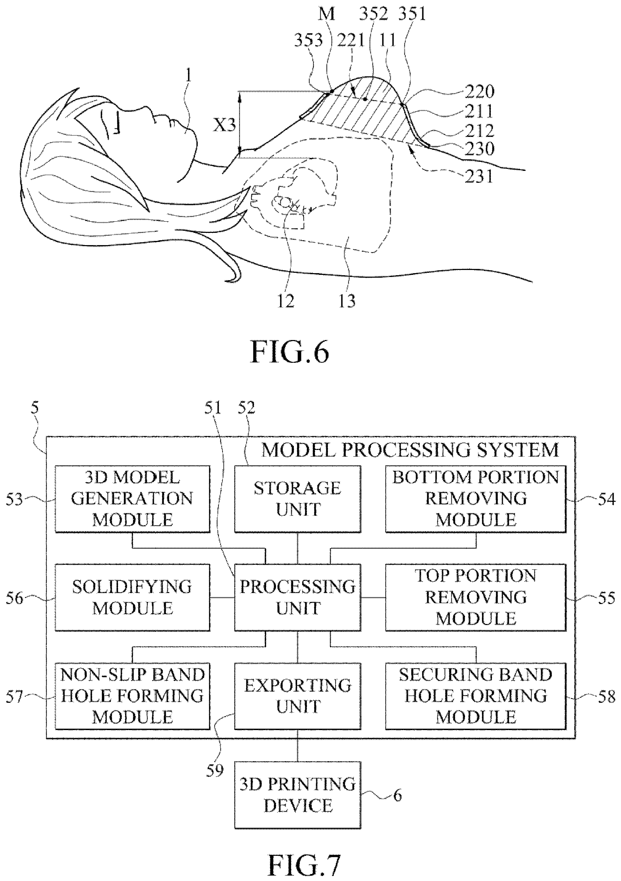

As shown in FIG. 2, the breast support device 2a further includes a plurality of securing band holes 222, 223, 224, and 225 for securing bands to go through. The securing band holes 222 and 223 are on the midsternal side 2121 of the base portion 212; the securing band holes 224 and 225 are on the midaxillary side 2122 of the base portion 212. In other words, while the breast support device 2a is properly worn on the left breast 11 of the female patient 1 (as shown in FIG. 6), the securing band holes 224 and 225 are adjacent to the outer side of the front chest or the torso of the female patient 1, and the securing band holes 222 and 223 are adjacent to the center of the front chest or torso.

In addition, the securing band holes 222 and 224 are adjacent to the upper side of the front chest; the securing band holes 223 and 225 are adjacent to the lower side of the front chest. A band, strap, buckle, or other secure members can pass through the securing band holes 222 and 224, similarly, another band, strap, buckle, or the like can pass through the securing band holes 223 and 225 for securing the breast support device 2a on the front chest.

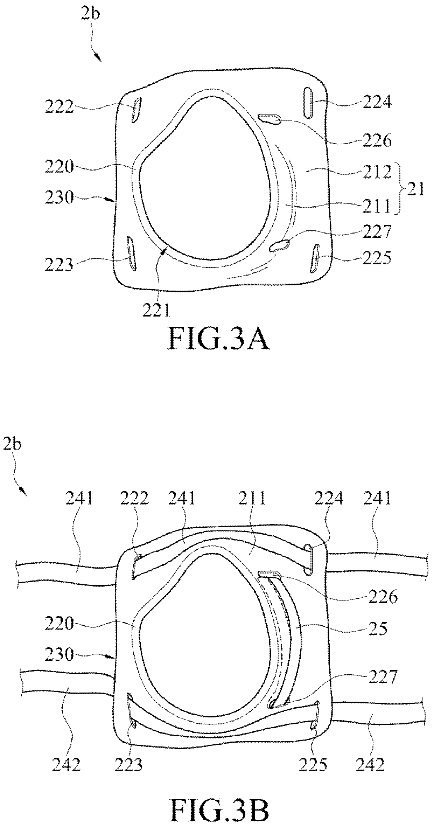

FIG. 3A illustrates a top views of a breast support device 2b according the present invention. The breast support device 2b is similar to the one illustrated in FIG. 2, except that it further comprises a plurality of anti-slip band holes 226 and 227 for anti-slip band to pass through. The anti-slip band holes 226 and 227 are located on the compression portion 211 of the breast support device 2b.

The two anti-slip band holes 226 and 227 are respectively adjacent to the securing band holes 224 and 225. The anti-slip band holes 226 and 227 are adjacent to the midaxillary side 2122 of the base portion 212. In addition, the anti-slip band holes 226 and 227 are close to the outer side (e.g., the left side) of the front chest while the breast support device 2b being properly worn on the left breast 11 of the female patient 1 (as shown in FIG. 6). A band, strap or the like may pass through the anti-slip band holes 226 and 227 to help securing the breast 11 on the beast supporting device 2b.

FIG. 3B illustrates a top views of the breast support device 2b the present invention. It has the same structure as the breast support device 2b shown in FIG. 3A except two additional securing bands 241, 242 and an anti-slip band 25 being putted on. The anti-slip band 25 helps securing female patient's breast 11 on the beast supporting device 2b.

In addition, while the breast support device 2b being worn by the female patient 1, one side of the patient's breast close to the midaxillary line is easily being pulled by the skin from the armpit, that side of the breast is easier to slip relative to the compression portion 211 of the breast support device 2b. Therefore, the anti-slip band 25 close to the midaxillary side 2122 is beneficial for preventing the breast of that side from slipping.

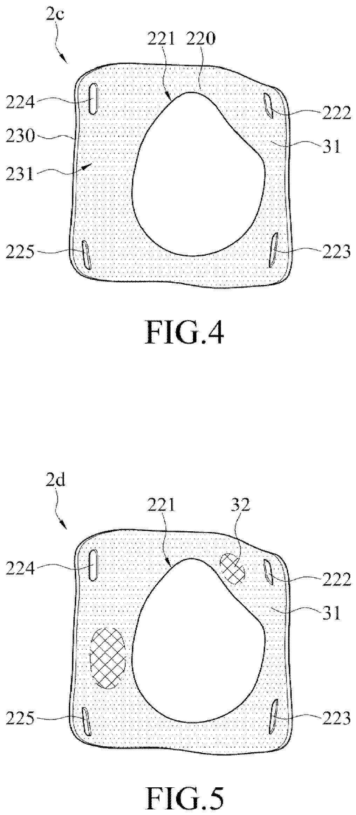

FIG. 4 illustrates a bottom view of a breast support device 2c according to the present invention. It illustrates a prospective view from the inner surface side of the breast support device 2c. The breast support device 2c has a similar structure as the previous described breast support device, except that the breast support device 2c further including a metal-nanoparticles-contained layer 31. The metal-nanoparticles-contained layer 31 is formed on an inner side or inner surface of the breast support device 2c. The inner surface is opposite with the outer surface of the body and would contact the female patient's breast while the breast support device 2c being worn on. The metal-nanoparticles-contained layer 31 is non-poisonous, it contains materials which can't be absorbed by human body and can be configured to sterilize the breast. In one embodiment, the metal-nanoparticles-contained layer 31 may include, for example, gold (Au), silver (Ag), copper (Cu), Titanium (Ti) and other metal or non-metal particles.

A patient with breast cancer may have to precede a series of radiation therapy treatments (e.g. more or less from ten to thirty treatments, depends on each patient's condition). Each treatment may cause discomfort on the patient's treated breast. For example, an inflammation in treated skin or breast tissues caused by the radiation treatment. The metal-nanoparticles-contained layer 31 can kill bacterium, facilitate healing of wounds caused by the inflammation, and therefore can ease the pain of the treated skin or breast tissues. The metal-nanoparticles-contained layer 31 may comprise materials such as silver capable of killing bacteria. The metal-nanoparticles-contained layer 31 may be a silver layer with silver compounds. In one preferred embodiment, the metal-nanoparticles-contained layer 31 may comprise materials such as gold, copper, aluminum, and titanium depending on different needs.

FIG. 5 illustrates a bottom view of a breast support device 2d according to the present invention. It illustrates a prospective view from the inner surface side of the breast support device 2c. The breast support device 2d is similar to the breast support device 2c illustrated in FIG. 4, except that the breast support device 2d further comprises a radiation enhancement layer 32. The radiation enhancement layer 32 is formed on the inner surface of the breast support device 2d and would contact the breast of the female patient.

The radiation enhancement layer 32 may include materials which is the same with or similar to that of metal-nanoparticles-contained layer 31. The radiation enhancement layer includes materials that can enhance power or performance of the radiation. The metal-nanoparticles-contained layer 31 and the radiation enhancement layer 32 have substantially the same thickness. The metal-nanoparticles-contained layer 31 is formed on areas of the inner surface of the breast support device 2d, where can cover the corresponding portions of the patient's breast with healthy tissues. In contrast, the radiation enhancement layer 32 is formed on areas of the inner surface of the breast support device 2d locating at areas with cancer cells in the patient's breast. In particular, the radiation enhancement layer 32 is configured to closer to cancel cells and the metal-nanoparticles-contained layer 31 is configured to cover the healthy cells, accordingly. Both the metal-nanoparticles-contained layer 31 and the radiation enhancement layer 32 are non-poisonous and are not absorbed by human body. Various pattern arrangements of the metal-nanoparticles-contained layer 31 and the radiation enhancement layer 32 can be configured depending on the positions of cancer cells and healthy tissues in patient's body.

FIG. 6 illustrates a female patient 1 wearing a breast support device 2a in a supine position according to the present invention. The breast support device 2a, depending on the location of the breast cancer, can be used to support either the left breast or right breast of a female patient. In one preferred embodiment, the breast support device 2a is used to support the left breast of the female patient 1.

As shown in FIG. 6, while the breast support device 2a (as shown in FIG. 2) being worn by a female patient 1, a breast 11 of the female patent 1 passes through the through aperture 221 of the breast support device 2a. The second cutting side 230 contacts the front chest of the female patient 1. The compression portion 211 compresses the bottom part of the breast 11 to extrude the top part of the breast 11 outside the breast support device 2a. The compression portion 211 can support the breast, withhold the distance between the patient's treated area of the breast to heart 12 and lung 13 and prevent it being affected by gravitational pulling. The bottom part of the breast 11 is a portion of the breast adjacent to the front chest.

The size of the through aperture 221 is usually less than that of the concave second opening 231.

Considering one of the breast support devices 2a, 2b, 2c, or 2d, is worn on the breast 11 of the female patient 1, a minimum distance between the reference point M of the breast 11 and the heart 12 is X3. Since breast support device 2a, 2b, 2c, or 2d can support the breast 11 well, even the female patient 11 is in the supine position, the distance X3 would be significantly greater than either the distance X1 or the distance X2 (FIG. 1). As a result, the portion with cancer cells inside the breast 11 needed to be treated would be fully exposed to the radiation beam and would maintain an essential distance away from the patient's heart 12 and lung 13.

FIG. 7 illustrates a block diagram of a model processing system 5 for forming a breast support device model according to the present invention. The breast support devices 2a, 2b, 2c, and 2d can be manufactured in 3D printing technique. A 3D printer can be used to form the breast support devices 2a, 2b, 2c, and 2d based upon a 3D model, e.g., the breast support device model. 3D printing refers to processes used to create a three-dimensional object in which material is jointed or solidified under computer control to create an object, with material being added together. Objects can be of almost any shape or geometry and typically are produced using digital model data from a 3D model or another electronic data source. 3D printing builds a 3-dimensional object from computer aided design (CAD) model, usually by adding material layer by layer.

The breast support device model is the 3D model data stored in a computer readable medium. The 3D model can be constructed by many polygons in a 3D coordinate system. Each of the polygons may be a triangle. The 3D printer can print the breast support device 2a, 2b, 2c, or 2d in 3D based on receiving and processing the breast support device model. Data of an initial 3D model for constructing the breast support device can be generated by a 3D scanning device and will discuss later. The model processing system 5 can process the initial 3D model to form a breast support device model for 3D printing.

As shown in FIG. 7, the model processing system 5 comprises a processing unit 51, a storage unit 52, a 3D model generation module 53, a bottom portion removing module 54, and a top portion removing module 55.

The storage unit 52, the 3D model generation module 53, the bottom removing portion 54, and the top portion removing module 55 are electrically connected to the processing unit 51.

The 3D model generation module 53 is used to generate a 3D model. The 3D model can be referred to an aforementioned initial 3D model, which is generated by a 3D scanning device and is further transmitted and stored in the storage unit 52. Namely, the image of the chest is taken by the 3D scanning device for constructing the 3D model. The processing unit 51 loads the initial 3D model and controls the 3D model generation module 53 to generate the 3D model based upon the initial 3D model. The 3D model is used for further processing to form a breast support device model.

The 3D model includes parts that can be referred to a corresponding female patient's breasts and chest contours, which are a bottom portion, a base portion, a compression portion, and a top portion. The compression portion is connected between the base portion and the top portion. The bottom portion is connected with and around the base portion. The compression portion and the top portion jointly form breasts contours, and the bottom portion and the base portion jointly form front chest contours. The base portion and the compression portion is referred to the base portion 212 and the compression 211 of the breast support devices 2a, 2b, 2c, and 2d.

The top portion removing module 55 removes the top portion to form a through aperture and a first cutting side. The top portion removing module 55 would define a cutting plane (not shown) in advance. The cutting plane crosses the 3D model between the compression portion and the top portion in a traverse arrangement. The cutting plane may be perpendicular to the axial direction, as discussed above, of the 3D model or has an angle relative to the axial direction between 90 degrees and 180 degrees. The top portion removing module 55 removes the top portion based upon the cutting plane. The first cutting side is coplanar, and the cutting plane overlaps the first cutting side.

In some embodiments, the 3D model further includes certain point (e.g., a cutting point) with a maximum curvature defined along the axial direction from the compression portion to the top portion, and the cutting plane crosses the cutting point. Alternatively, the 3D model comprises a transition region. The transition region is defined by a part of the compression portion and a part of the top portion adjacent to each other.

A curvature of any point on the transition region defined along the axial direction from the compression portion to the top portion is greater than that of any point on the compression portion and the top portion outside the transition region defined along the axial direction.

In some embodiments, at least one point on the transition region has a maximum curvature along the axial direction comparing to any points on the compression portion and the top portion. Curvatures of points on the transition region along the axial direction are equal to the maximum curvature or no less than 90% of the maximum curvature. For example, a point on the transition region may have a minimum curvature along the axial direction comparing to any points on the transition region along the axial direction, and the minimum curvature of the point on the transition region is 10% less than the maximum curvature.

Additionally, the model processing system 5 further comprises a bottom portion removing module 54, a solidifying module 56, an anti-slip band hole forming module 57, a securing band hole forming module 58, and an exporting unit 59, each of which is electrically connected to and controlled by the processing unit 51.

The bottom portion removing module 54 is used to process the 3D model and to remove the bottom portion to form a second cutting side and a second cutting opening. The second cutting side is on a bottom of the base portion away from the compression portion, and the second cutting opening is surrounded by the second cutting side. The second cutting side and a second cutting opening can be referred to the second cutting side 230 and the second cutting opening 231 of the breast support devices 2a, 2b, 2c, and 2d.

The solidifying module 56 is used to process the 3D model and to solidify the compression portion and the base portion in an extruding manner. In an embodiment, the compression portion and the base portion are extruded along a normal direction of an inner surface of the 3D model by the solidifying module 56 to form a designed thickness. The designed thickness can be referred to an actual thickness of the compression portion 211 and the base portion 212 of the breast support devices 2a, 2b, 2c, and 2d. The designed thickness and the actual thickness may be ranged from 3 mm to 10 mm. That is to say, the solidifying module 56 has the compression portion and the base portion of the 3D model extruded from 0 mm (an original position) to -3mm to -10 mm. The negative sign means that the direction of the extruding faces towards the inside of the 3D model.

In some embodiments, the solidifying module 56 has the compression portion and the base portion of the 3D model extruded from -0.1 cm (an offset position) to -0.6 cm. In such case, the solidifying module 56 has the compression portion and the base portion offset (or shrunk) towards inside by 0.1 cm in advance, and then has the shrunk compression portion and base portion extruded inside to form a 3 mm to 10 mm thickness. Under the circumstances, the breast support devices 2a, 2b, 2c, and 2d made by the 3D model would be smaller. The intentionally smaller breast support devices 2a, 2b, 2c, and 2d are suitable for certain patients. For example, a patient has received breast conserving surgery (BCS), and a part of her breast (tissues inside the breast) has been removed; therefore, her breast requiring further radiation therapy may have a looser structure. The intentionally designed smaller breast support devices 2a, 2b, 2c, and 2d are benefit to support the breast with looser structure.

The anti-slip band hole forming module 57 is used to process the 3D model and to form a plurality of anti-slip band holes on the compression portion for an anti-slip band to pass through. The anti-slip band holes of the 3D model can be referred to the anti-slip band holes 226 and 227 of the breast support devices 2a, 2b, 2c, and 2d.

The securing band hole forming module 58 is used to process the 3D model and to form a plurality of securing band holes on the base portion for a securing band to pass through. The securing band holes can be referred to the securing band holes 222, 223, 224, and 225 of the breast support devices 2a, 2b, 2c, and 2d.

After the 3D model is processed by the model processing system 5, the processed 3D model (i.e., the breast support device model) would be analogous to the breast support device 2a, 2b, 2c, or 2d in geometric structure and dimension. The processing unit 51 can have the processed 3D model stored in the storage unit 52. The exporting unit 59 is signally connected to a 3D printing device 6 capable of performing 3D printing. The processing unit 51 can have the processed 3D model exported to the 3D printing device 6 via the exporting unit 59 for 3D printing to form the breast support device 2a, 2b, 2c, or 2d.

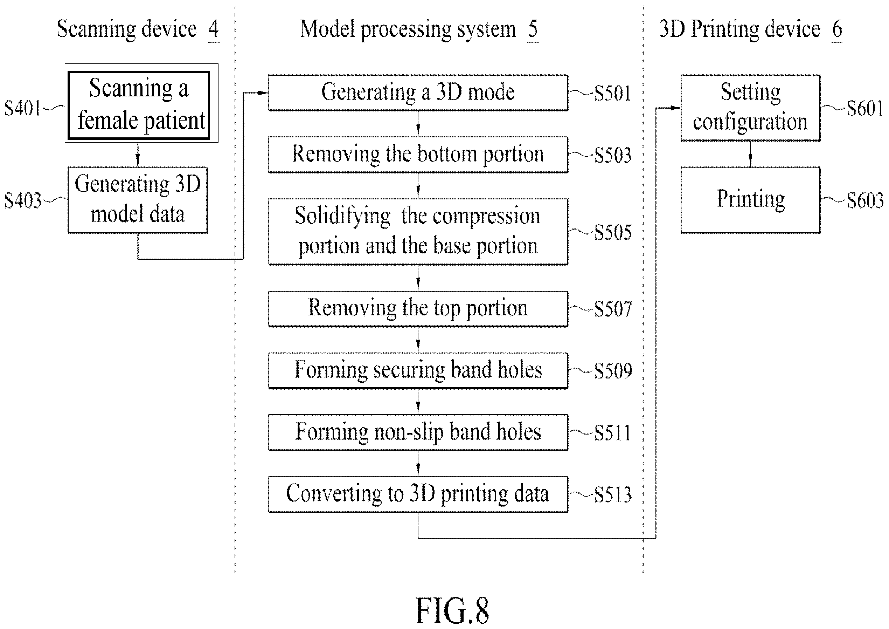

The sequential process by which the breast support device can be fabricated is represented by FIG. 8, according to the present invention.

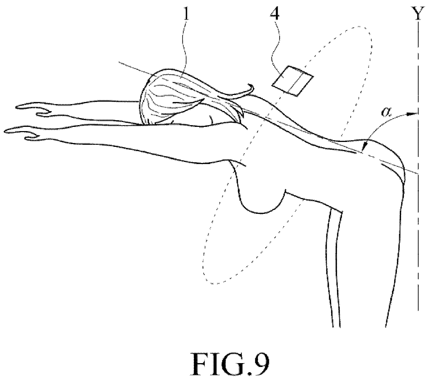

In an embodiment, in step S401, the female patient's breast and chest is scanned by a doctor (or use an instrument to) with a 3D scanner in a predetermined posture to form an initial scanned data (breast and/or chest image). The predetermined posture as illustrated in FIG. 9, the female patient 1 may take a position, e.g., her torso being hunched forward with her arms stretching over her head, such that the back of her torso forms an angle α to a vertical axis Y, where α may range from substantially 30 degrees to substantially 90 degrees. A scanning device 4, e.g., a 3D scanner, is used to move around the entire chest by the doctor to collect 3D image information of the chest (or the torso).

In step S403, the scanning device 4 generates 3D model data of the patient's chest to form an initial 3D model. The 3D model data of the initial 3D model can be transferred to the model processing system 5 for further processing.

In step S501, a 3D model is constructed based on the 3D model data generated by the scanning device 4. In addition, the initial 3D model may be modified by the model processing system 5 to eliminate shadow or dark portions or breaches to form the 3D model.

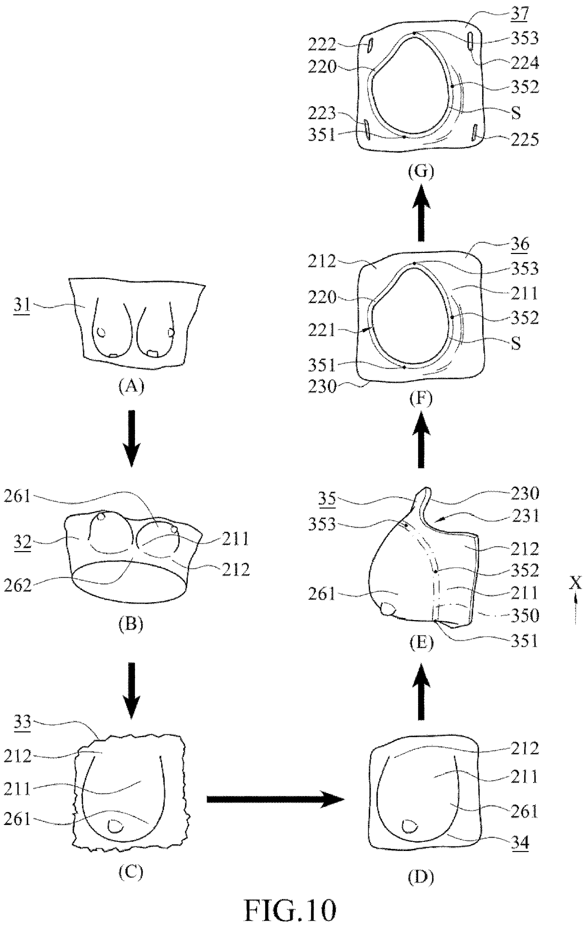

In step S503, the bottom portion removing module 54 of the model processing system 5 shown in FIG. 7 removes the bottom portion, the right breast and the rear chest of the female patient's 3D model to form an initial breast supporting 3D model 33(FIG. 10C). In some embodiments, the initial breast supporting 3D model is further processed to form a smoothed or trimmed 3D model 34 (FIG. 10D).

In step S505, the solidifying module 56 solidifies the top portion, the compression portion, and the base portion in an extruding manner to form a solidified breast supporting 3D model 35 (FIG. 10E). In another embodiment, the top portion may be removed in advance, and then the compression portion and the base portion can be solidified. The top portion, the compression portion, and the base portion are extruded along a normal direction of an inner surface of the solidified breast supporting 3D model 35 (FIG. 10E) to form a desired thickness. In addition, the solidified breast supporting 3D model 35 is formed with the second cutting side and the second cutting opening. As discussed above, the second cutting side is on the bottom of the base portion away from the compression portion, and the second cutting opening is surrounded by the second cutting side.

In step S507, the top portion removing module 55 removes the top portion based upon the transition region 350 to form a breast supporting 3D model 36 (FIG. 10F) with through aperture.

In step S509, the securing band hole forming module 58 forms the securing band holes on the base portion to form the finalized breast supporting 3D model 37 (FIG. 10G). In step S511, the anti-slip band hole forming module 57 may also form the anti-slip band holes on the compression portion.

In step S513, the exporting unit 59 coverts the processed 3D model (the breast support device model such as the 3D models 36 or 37) into certain 3D printing data that the 3D printing device 6 can access and print. The exporting unit 59 transmits the converted 3D printing data to the 3D printing device 6.

It shall be understood that the orders of the steps as the above discussion is merely for illustration and is not to limit the scope of the present invention. The steps of forming a breast support device model can be varied in different embodiments depending upon different situations.

In step S601, the 3D printing device 6 receives the 3D printing data (chest or breast 3D image) and sets configuration of the 3D printing data for printing. For example, the configuration may include patient's physical parameters, posture, and position that are related to the printing process and a working space.

In an embodiment, the coplanar first cutting side 220 may be aligned with an initial plane in the working space of the 3D printing device 6 from which the printing process initiates. In such case, a breast support device can be printed in 3D from the first cutting side 220 to the second cutting side 230 (FIG. 10F). A plurality of support columns automatically are added by the 3D printing device 6, which are arranged between the initial plane and the outer surface 211s of the compression portion 211, and the base portion 212 can be printed in the printing process of the breast support device. The support columns provide supports for the compression portion 211 and the base portion 212 during the printing process, which can be removed after completing the breast support device.

In step S603, the 3D printing device 6 starts to print a breast support device based upon 3D printing data relative to a breast support device model.

FIG. 10A-G illustrate the breast supporting device model at various stages, which are corresponded to steps illustrated in FIG.8 according to the present invention.

FIG. 10A shows an initial 3D model 31 of the female patient's chest according to the scanned 3D image information mentioned in step S403 (FIG. 8).

FIG. 10B illustrates a 3D model of a female patient's chest and breasts 32 generated by the 3D model generation module 53 based on the 3D model data that mentioned in step S501 (FIG. 8). The 3D model 32 comprises a top portion 261, a bottom portion 262, a compression portion 211, and a base portion 212. The bottom portion 262 and the base portion 212 jointly form contours that fit to a female patient's chest. The compression portion 211 and the top portion 261 jointly form contours that fit to the female patient's breast.

FIG. 10C illustrates an initial breast supporting 3D model 33 after removing the bottom portion 262 as well as the right breast and the rear chest of the previous 3D model 32, which is described in step S503 (FIG. 8). As a result, the initial breast supporting 3D model 33 includes only the base portion 212, the compression portion 211, and the top portion 261.

FIG. 10D demonstrates the base portion 212 of the initial breast supporting 3D model 33 that is further processed to form a smoother initial breast supporting 3D model 34.

FIG. 10E illustrates a solidified breast supporting 3D model 35 after solidifying the top portion 261, the compression portion 211, and the base portion 212 that mentioned in step S505 (FIG. 8) by the solidifying module 56 (FIG.7).

FIG. 10F illustrates a breast supporting 3D model with through aperture 36 formed by removing the top portion 261 based on a defined transition region 350 as mentioned in step S507(FIG. 8). In one preferred embodiment, the top portion 261 is removed along a plane crossing the transition region 350. The cutting plane is defined by three points 315, 352, and 353.

FIG. 10G illustrates a finalized breast supporting 3D model 37 with the formation of securing band holes 222, 223, 224, and 225 on the base portion, as mentioned in step S509 (FIG. 8).

In summary, the breast support device 2a is constructed from the 3D model as demonstrated in FIG. 10. The 3D model forms a shape like a portion of a breast cup having a concave inner surface, where the concave inner surface has contours that fit over patient's breast as patient been in a predetermined posture (shown in FIG. 9). A through aperture is formed by removing part of the breast cup to receive a portion of the female patient's breast through.

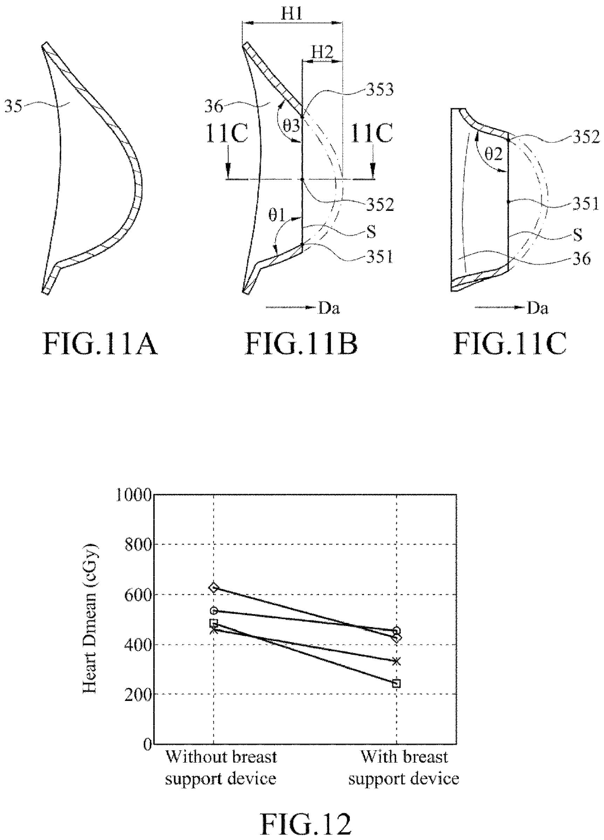

FIG. 11A-C illustrate cross-sectional views of a 3D model being processed according to an embodiment of the present invention. FIG. 11A is a cross-sectional view of the solidified breast supporting 3D model 35 as shown in FIG. 10(E). FIG. 11B is a cross-sectional view of the breast supporting 3D model with through aperture 36 as shown in FIG. 10(F). FIG. 11C is another cross-sectional view of the breast supporting 3D model with through aperture 36 across line 11C-11C of FIG. 11B. The breast supporting 3D model with through aperture 36 can be formed by removing a part (which is divided by dotted lines and can be referred the top portion 261) of the solidified breast supporting 3D model 35 as shown in FIG. 10(E). The breast supporting 3D model with through aperture 36 has a height H1 defined along an axial direction (i.e., the direction Da in FIGS. 11B-11C). The height H1 equals to the height of the compression portion 211 plus the top portion 261 along the axial direction Da. The removed part (i.e., the top portion 261) has a height H2 defined along the axial direction Da. A ratio of the height H2 to the height H1 is substantially equal to or greater than 1/4.

An angle θ1 defined by a lower side of the compression portion 211 relative to the cutting plane S ranges from substantially 80 degrees to substantially 170 degrees. An angle θ2 is defined by a lateral side (the side close to the midaxillary side 2122) of the compression portion 211 relative to the cutting plane S ranging from substantially 80 to 170 degrees. An angle θ3 is defined by an upper side of the compression portion 211 relative to the cutting plane S ranging from substantially 80 to 170 degrees. The removed portion (i.e., the top portion 261) of the solidified breast supporting 3D model 35 in FIGS. 11B and 11C is shown by dotted lines.

FIG. 12 illustrates a line charts showing the doses absorbed by female patient's hearts, each line represents two data points of individual patient with and without breast support devices under radiation treatments according to the present invention. The line chart shows an actual statistics collected by the inventors. These data collected the radiation doses absorbed by the hearts of the female patients after receiving the radiation therapy for breast cancer. Four lines respectively represent the radiation doses absorbed by the hearts of four individual female patients. Each line has two end points, the left represents the radiation dose absorbed by the heart of one of the female patients after receiving the radiation therapy without wearing any breast support device, and the right represents the radiation dose absorbed by the heart of the same female patient after receiving the radiation therapy with the breast support device on. All of the four lines show consistently that the radiation dose absorbed by the heart of a female patient with a breast support device on under the radiation therapy is significantly lesser than those patients without wearing any breast support device.

Concisely, the breast support device according to embodiments of the present invention is beneficial for protecting healthy organs and tissues from radiation under the radiation therapy for breast cancer. In addition, the method and the system for forming a breast support device model according to embodiments of the present invention provide easily and conveniently measures to manufacture the breast support device.

As will be understood by persons skilled in the art, the foregoing preferred embodiment of the present invention illustrates the present invention rather than limiting the present invention. Having described the invention in connection with a preferred embodiment, modifications will be suggested to those skilled in the art. Thus, the invention is not to be limited to this embodiment, but rather the invention is intended to cover various modifications and similar arrangements included within the spirit and scope of the appended claims, the scope of which should be accorded the broadest interpretation, thereby encompassing all such modifications and similar structures. While the preferred embodiment of the invention has been illustrated and described, it will be appreciated that various changes can be made without departing from the spirit and scope of the invention.

## Claims

1. A breast support device for radiation therapy, **characterized by** comprising:
a cuplike body having a base portion, a compression portion connected to the base portion to form a concave inner surface, wherein the concave inner surface has contours that fit over a female patient's breast; and
wherein the compression portion includes a through aperture to receive a portion of the female patient's breast through the through aperture, wherein the through aperture has a contour curve defined by a cutting plane and the contours of the female patient's breast.

2. The breast support device for radiation therapy of claim 1, **characterized in that** the contours are 3D image of the female patient's breast taken at a position in which the female patient's torso is hunched forward with her arm stretching over her head, such that the back of her torso forms an angle α to a vertical axis Y.

3. The breast support device for radiation therapy of claim 2, **characterized in that** the angle α is ranged from 30 degrees to 90 degrees.

4. The system for forming a breast support device model of claim 2, **characterized in that** the vertical axis Y is parallel to the normal direction of the floor plane.

5. The breast support device for radiation therapy of claim 1, **characterized in that** the concave inner surface of the cuplike body further comprises contours that fit over a portion of the female patient's chest adjacent to the breast.

6. The breast support device for radiation therapy of claim 1, **characterized in that** the cutting plane for forming the through aperture is a plane parallel to or with an angle θ to the axial axis of the compression portion of the cuplike body, the angle θ is ranged from 10 degrees to 90 degrees.

7. The breast support device for radiation therapy of claim 1, **characterized in that** the cuplike body further comprises a plurality of anti-slip band holes located at the compression portion for assisting the support of the breast support device by passing through bands.

8. The breast support device for radiation therapy of claim 1, **characterized in that** the cuplike body further comprises a plurality of securing band holes located at the base portion for securing the breast support device on patient's torso by passing through securing bands.

9. The breast support device for radiation therapy of claim 1, **characterized in that** the cuplike body further comprises a metal layer on the inner surface for blocking radiation.

10. breast support device for radiation therapy of claim 1, **characterized in that** the cuplike body further comprises a radiation enhancement layer on the inner surface for enhancing radiation.

11. A breast support device for radiation therapy, **characterized by** comprising:
a cuplike body having a base portion, a compression portion connected to the base portion to form a concave inner surface, wherein the compression portion is extended from the base portion, the concave inner surface has contours that fit over the female patient's breast and has a radiation enhancement layer for enhancing radiation; and
wherein the compression portion includes a through aperture to receive a portion of the female patient's breast through the through aperture.

12. The breast support device of claim 11, **characterized in that** the contours are 3D image of the female patient's breast taken at a position in which the female patient's torso is hunched forward with her arm stretching over her head, such that the back of her torso forms an angle α to a vertical axis Y, wherein the angle α is ranged from 30 degrees to 90 degrees, the vertical axis Y is parallel to the normal direction of the floor plane.

13. The breast support device for radiation therapy of claim 11, **characterized in that** the concave inner surface of the cuplike body further comprises contours that fit over a portion of the female patient's chest adjacent to the breast.

14. The breast support device for radiation therapy of claim 11, **characterized in that** the cutting plane for forming the through aperture is a plane parallel to or with an angle θ to the axial axis of the compression portion of the cuplike body, the angle θ can be ranged from 10 degrees to 90 degrees.

15. The breast support device for radiation therapy of claim 11, **characterized in that** the through aperture has a contour curve defined by a cutting plane and the contours of the patient's breast.
